# EUROPEAN PATENT SPECIFICATION

(11) **EP 1 490 098 B1**
(45) Date of publication and mention of the grant of the patent: **17.03.2010**
(21) Application number: 03707982.9
(22) Date of filing: 25.03.2003
(51) Int. Cl.: A61K 39/00, A61K 39/39, A61P 35/00

(54) **CHEMOTHERAPEUTIC AGENTS AS ANTI-CANCER VACCINE ADJUVANTS AND THERAPEUTIC METHODS THEREOF**
THERAPEUTISCHE MITTEL ALS ANTIKREBS IMPFSTOFFADJUVANTIEN UND DEREN THERAPEUTISCHE VERFAHREN
AGENTS CHIMIOTHERAPEUTIQES UTILISES EN TANT QU'ADJUVANTS VACCINAUX CONTRE LE CANCER ET PROCEDES THERAPEUTIQUES CORRESPONDANTS

(30) Priority: 25.03.2002 US 366531 P
(43) Date of publication of application: 29.12.2004
(73) Proprietor: Technologie Biolactis Inc., Laval, QC H7V 5B7 (CA)
(72) Inventor: SIMARD, Eric, Laval, Québec H7L 5Z7 (CA); GOYETTE, Philippe, Montréal, Québec H2L 2T1 (CA); LEMIEUX, Pierre, Ste-Thérèse, Québec J7E 4Z2 (CA)
(74) Representative: Jacob, Reuben Ellis
(86) International application number: PCT/CA2003/000434
(87) International publication number: WO 2003/080111

(56) References cited:
- WO-A-98/16246
- US-A1- 2001 038 841
- DATABASE MEDLINE [Online] US NATIONAL LIBRARY OF MEDICINE (NLM), BETHESDA, MD, US; February 2001 (2001-02), EMENS L A ET AL: "Chemotherapy: friend or foe to cancer vaccines?" XP002254169 Database accession no. NLM11249735 & CURRENT OPINION IN MOLECULAR THERAPEUTICS. FEB 2001, vol. 3, no. 1, February 2001 (2001-02), pages 77-84, ISSN: 1464-8431
- DATABASE BIOSIS [Online] BIOSCIENCES INFORMATION SERVICE, PHILADELPHIA, PA, US; 16 November 2000 (2000-11-16), REECE D E ET AL: "Use of the anti-idiotype (ID) antibody (AB) vaccine 11D10 (TRIAB) in patients (PTS) with metastatic breast cancer (MBC) undergoing autologous stem cell transplantation (ASCT)." XP002254170 Database accession no. PREV200100317653 & BLOOD, vol. 96, no. 11 Part 1, 16 November 2000 (2000-11-16), page 844a, 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- RADCLIFF FIONA J ET AL: "Mobilization of dendritic cells in cancer patients treated with granulocyte colony-stimulating factor and chemotherapy." BRITISH JOURNAL OF HAEMATOLOGY OCTOBER, 2002, vol. 119, no. 1, October 2002 (2002-10), pages 204-211, XP002254168 & ISSN: 0007-1048
- Nigam, A. et al (1998) International Journal of Oncology 12:161-170

## Description

### BACKGROUND OF THE INVENTION

### (a) Field of the Invention

The present invention relates essentially to novel anti-cancer vaccine adjuvant composed of immunomodulator chemotherapeutic agents and uses thereof.

### (b) Description of Prior Art

It is well accepted that vaccines have contributed to improve quality of life of mankind and livestock and currently many different vaccines are still being developed to treat, cure, or prevent disease that can benefit from an immune response raised against an unwanted antigenic expression. Diseases like infectious diseases, autoimmune diseases, allergies, and cancers can benefit from the use of an efficient vaccine. For instance, as a tumor grows in size, it typically becomes more refractory to most chemotherapies. Accordingly, many tumor eradication procedures include radiotherapy or a surgical debulking step to decrease the mass of the tumor prior to the administration of anti-neoplastic agents. However, radiotherapy and debulking do not always result in tumor eradication, even when combined with powerful chemotherapeutic agents. Accordingly, there is a need in the art for new treatments that target both proliferating and non-proliferating cancer cells for the treatment of malignancies. Cancer vaccines have shown promise so far and with the examples given in this invention those may reach another level of efficacy with the use of chemotherapeutic agent used as adjuvants. Recent advances in the understanding of immune function with regard to the generation of a potent antitumor response have resulted in a renewed interest in cancer vaccines and point to a role for immunotherapy in the treatment of cancer.

Cancer vaccines are on the threshold of taking their place alongside the more traditional and conventional cancer treatment modalities of surgery, radiation therapy and chemotherapy. The toxicology and immunopharmacology of therapeutic cancer vaccines, particularly those that secrete granulocyte macrophage colony stimulating factor (GM-CSF), are currently under active clinical investigation (Cell Genesys). GM-CSF is used as an adjuvant to help function of antigen presentation. This kind of vaccine however requires multiple steps like expansion of cancer cell in culture, transfection with GM-CSF, inactivation with irradiation before the reinoculation. With this kind of vaccine, a priming step of the patient may be enhancing the therapeutic effect. Interestingly, drugs traditionally used for tumor cytoreduction can have both positive and negative effects on host immunity. Exploration of the potential pharmacodynamic interactions of antineoplastic drugs administered systemically along with GM-CSF-secreting vaccines has revealed that low doses of some chemotherapeutics can augment the antitumor immunity induced by GM-CSF-secreting vaccines.

Chemotherapeutics are the mainstay of the majority of antitumor treatment strategies. These agents are usually administered at or near maximum tolerated doses resulting in frequent dramatic toxicities that compromise the quality of life and the immune response towards microbial pathogens. A number of observations suggest that low-dose treatment with chemotherapeutics is sometimes equal or even superior to high-dose chemotherapy. The efficacy of low-dose chemotherapy can be at least partly explained by the regulation of the antitumor immune response. The immunomodulatory effects of some chemotherapeutics might be further potentiated by combinations with selected biological response modifiers such as recombinant cytokines (IL-2, TNF, IL-12 and others). The effectiveness of such treatment combinations have already proven effective in preclinical animal models. However, the efficacy in humans is still to be demonstrated in rationally designed clinical trials (Zagozdzon et al. Int J Oncol 2001 Feb; 18(2):417-24).

For instance, it was observed that cyclophosphamide, paclitaxel, and doxorubicin, when given in a defined sequence either before or after the whole-cell vaccine and by a different route of administration with a GM-CSF-secreting, neu-expressing whole-cell vaccine, enhanced the vaccine's potential to delay tumor growth in neu transgenic mice. In addition, it was showed that these drugs mediate their effects by enhancing the efficacy of the vaccine rather than via a direct cytolytic effect on cancer cells. Furthermore, paclitaxel and cyclophosphamide appear to amplify the T helper 1 neu-specific T-cell response. These findings suggest that the combined treatment with immune-modulating doses of chemotherapy and the GM-CSF-secreting neu vaccine can overcome immune tolerance and induce an antigen-specific antitumor immune response (Machiels et al. Cancer Res 2001 May 1;61 (9):3689-97). The approach described above is unfortunately complex and requires a timing allowing the best performance possible of the whole-cell vaccine. Thus, there is a need to simplify and to increase the efficacy of such approach. Chemotherapeutic agents have been shown to stimulate immune responses against antigens (Bystryn US patent 6,338,853). For instance, in some clinical trials it was found that antibody and/or cellular immune responses to melanoma were induced more frequently in Stage II (69% of 36 patients) than in Stage III (53% of 19 patients) disease. The ability of different immunization schedules, alum or pretreatment with low dose cyclophosphamide, to potentiate immunogenicity was compared after 2 months of immunization. Bi-weekly immunization with a fixed intermediate dose of vaccine was more immunogenic than weekly immunization with escalating vaccine doses. Alum increased slightly the intensity of cellular responses while pretreatment with cyclophosphamide augmented slightly both the incidence and intensity of cellular immune responses. There was a reciprocal relationship between the induction of humoral and cellular immune responses. The most effective immunization schedule consisted of pretreatment with cyclophosphamide which augmented antibody and/or cellular immune responses to melanoma in 83% of patients. In addition, recently, paclitaxel, another well known and widely used chemotherapeutic agent was found to trigger the same signal transduction pathways than LPS, and to be also a strong inducer of multiple immune responses (Byrd-Leifer et al. Eur J Immunol 2001 Aug;31 (8):2448-57).

In Emens; L.A. et al ((2001) Current Opinion in Molecular Therapeutics 3:77-84) and Nigam, A et al, ((1998) Int. J. Oncol. 12:161-170), the pharmacodynamic interactions between antineoplastic drugs and GM-CSF-secreting vaccines are discussed. However, these documents do not describe a single composition that can contain both of these agents.

These observations are of interest but schedules and the timing of treatment are complicated since the chemotherapetic agents have to be injected either before or after the antigens. Furthermore, chemotherapeutic agents have to be administered at the appropriate concentration in order to promote the stimulation of the immune system rather than a systemic immunosuppression normally associated with the use of chemotherapeutic agents.

Despite the development of Monophosphoryl lipid A (MPL), the latter has shown limited efficacy in clinical trials on cancer vaccines and other types of vaccines. Thus, there is still a need for novel and more efficacious adjuvants that may enhance further immunogenicity of conventional vaccines but especially of new-generation vaccines (including recombinant subunit, peptide and protein-based vaccines, DNA vaccines, whole-cell vaccine, mucosal vaccines) that are less immunogenic. GM-CSF is being used as a molecular adjuvant (Cell Genesys) to enhance immunogenicity of whole-cell vaccines against cancer. The GM-CSF gene is transfected into the cells before being expanded in culture and be inoculated.

Currently, aluminum salts and MF59 are the only vaccine adjuvants approved for human use. Aluminum salts used as adjuvants are now suspected to be the culprit of the Gulf war syndrome which translates to a chronic fatigue due to an accumulation of aluminum crystals in the muscle leading to a constant immunostimulation and eventually to depletion of competent immune cells. With the development of new-generation vaccines (including recombinant subunit, DNA vaccines, whole-cell vaccine, mucosal vaccines) that are less immunogenic, the search for more potent vaccine adjuvants has intensified. Of the novel compounds recently evaluated in human trials, immunostimulatory molecules such as the lipopolysaccharide derived MPL (Corixa) and the saponin derivative QS21 appear most promising, although doubts have been raised as to their safety in humans. Preclinical work with particulate adjuvants, such as the MF59 microemulsion and lipid-particle immune-stimulating complexes (Iscoms), suggest that these molecules are potent elicitors of humoral and cellular immune responses. In addition, preclinical data on CpG oligonucleotides appear to be encouraging, particularly with respect to their ability to selectively manipulate immune responses. While all these adjuvants show promise, further development of more potent adjuvants may allow vaccines to be used as therapeutic, rather than prophylactic agents.

It would be highly desirable to be provided with an anti-cancer vaccine composition comprising immunomodulator chemotherapeutic agents as adjuvant to elicit an immune response against cancer in a patient.

### SUMMARY OF THE INVENTION

In accordance with the present invention there is provided an anti-cancer vaccine composition comprising an antigen in association with an effective amount of at least one immunomodulator chemotherapeutic adjuvant eliciting an immune response in a patient and a pharmaceutically acceptable carrier.

The anti-cancer vaccine in accordance with a preferred embodiment of the present invention, wherein the vaccine is selected from the group consisting of whole-cell vaccine, DNA vaccine, peptide-based vaccine, protein-based vaccine and attenuated organism vaccine.

The anti-cancer vaccine in accordance with a preferred embodiment of the present invention, wherein the antigen is inactivated tumor cells more preferably wherein the tumor cells being inactivated by the chemotherapeutic agent or wherein said tumor cells are inactivated by radiotherapy and/or chemotherapy.

The anti-cancer vaccine in accordance with another embodiment of the present invention, wherein the tumor cells are inactivated by radiotherapy.

The anti-cancer vaccine in accordance with a preferred embodiment of the present invention, wherein said vaccine is eliciting an immune response against a cancer selected from the group consisting of basal cell carcinoma, bladder cancer, bone cancer, brain cancer, CNS cancer, breast cancer, cervical cancer, colon cancer, rectum cancer, connective tissue cancer, esophageal cancer, eye cancer, kidney cancer, larynx cancer, liver cancer, lung cancer, Hodgkin's lymphoma, non-Hodgkin's lymphoma, melanoma, myeloma, leukemia, oral cavity cancer, ovarian cancer, pancreatic cancer, prostate cancer, rhabdomyosarcoma, skin cancer, stomach cancer, testicular cancer, neoplasia and uterine cancer.

The anti-cancer vaccine of a preferred embodiment of the present invention, wherein the vaccine is eliciting an immune response against a cancer selected from the group consisting of bladder cancer, prostate cancer, melanoma, breast cancer, colon cancer, lung cancer, liver cancer, lymphoma, myelona, leukemia and ovarian cancer.

In the anti-cancer vaccine of the present invention, the chemotherapeutic adjuvant is a taxane.

In the anti-cancer vaccine of a preferred embodiment of the present invention, the chemotherapeutic adjuvant is Paclitaxel.

The anti-cancer vaccine in accordance with another embodiment of the present invention, further comprising a therapeutically effective amount of a therapeutic agent.

In the anti-cancer vaccine in accordance with another embodiment of the present invention, the therapeutic agent is selected from the group consisting of cytokine, antibody, systemic chemotherapeutic agent, biological response modifier and hormones.

In the anti-cancer vaccine in accordance with another embodiment of the present invention, the antibody is a monoclonal antibody.

In the anti-cancer vaccine in accordance with another embodiment of the present invention, the biological response modifier is selected from the group consisting of interferon and lymphokine.

In the anti-cancer vaccine in accordance with another embodiment of the present invention, the lymphokine is IL-2.

The anti-cancer vaccine in accordance with another embodiment of the present invention, further comprising a non-chemotherapeutic adjuvant selected from the group consisting of polysaccharides, CpG nucleic acids, MF59, SAF, MPL and QS21.

The anti-cancer vaccine in accordance with another embodiment of the present invention, further comprising a non-chemotherapeutic adjuvant selected from the group consisting of adjuvants that create a local reservoir of drug, adjuvants that stimulate the immune system, mucosal adjuvants, phosphazene and *Leishmania* elongation factor.

For the purpose of the present invention the following terms are defined below.

The term "antigen" is intended to mean any molecule or cellular organism able to trigger an immune response from a patient and includes, without limitation, inactivated tumor cells.

The term "vaccine" is intended to mean a recombinant subunit, peptide vaccine, protein-based vaccines, DNA vaccines, whole-cell vaccine, mucosal vaccines, attenuated and live organism vaccines.

The term "adjuvant" is intended to mean a substance or substances that help eliciting an immune response against antigen by a patient.

The term "immunomodulator chemotherapeutic adjuvant" is intended to mean any chemotherapeutic agent capable of acting as an adjuvant as previously defined, in that they can elicit an immune response probably due to their structural effect on the cell's cytoskeleton and/or microtubules.

### BRIEF DESCRIPTION OF THE DRAWINGS

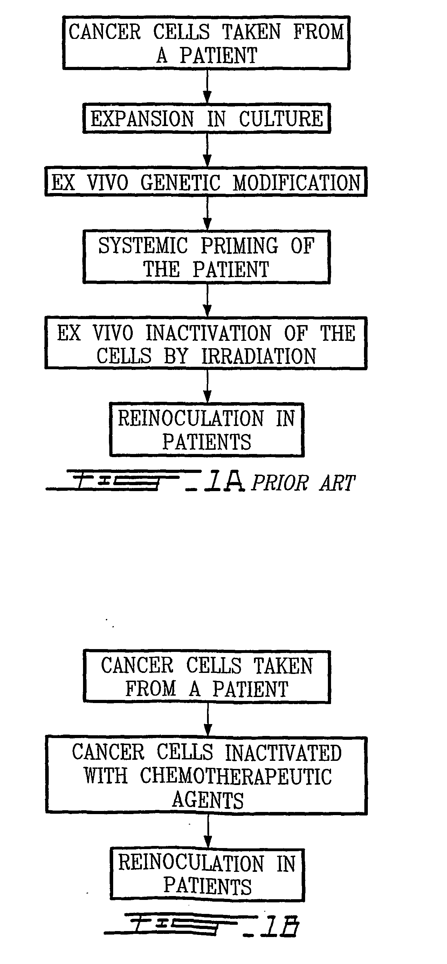

Figs. 1A and 1B illustrate a comparison between a whole-cell vaccine of the prior art and the whole-cell vaccine as described in one embodiment of the present invention.

### DETAILED DESCRIPTION OF THE INVENTION

In accordance with the present invention, there is provided an anti-cancer vaccine composition comprising chemotherapeutic agents as adjuvant to elicit an improved immune response in a patient.

What is being described in this application is the use of immunomodulator chemotherapeutic agents as adjuvants for anti-cancer vaccine to elicit immune response to a patient. It was found that it is possible to enhance immunogenicity of a vaccine by combining directly the immunomodulator chemotherapeutic agents with the vaccine in one single administration. The intramuscular route of administration is preferred but administration can also be done mucosally, orally, intranasally, intratracheally by inhalation, ocularlly, vaginally, intravenously, intra-arterially and rectally.

As shown in Example I, paclitaxel triggers the induction of MCP-1, a chemokine known to recruit dendritic cells (APC) at the injection site, a critical event for the induction of immune responses. Furthermore, Examples II and IV show that local injection of high concentration of paclitaxel is possible and do not provoke local toxicity that could be leading to immunosuppression. The fact that antibodies could be raised against an antigen in presence of paclitaxel is a sign that paclitaxel does not impede with the development of a normal immune reaction. In fact, it even helped to raise the immune response against a protein that always needs to be adjuvanted in order to be immunogenic. This is unexpected since chemotherapeutic agents are associated with immunosupression due to death of immune cell. In fact, the collateral presentation of the whole-cell vaccine helps to break tolerance against cancer cells as shown in Examples III and V.

This unexpected observation lead to the immunological rationale for testing immune-modulating doses of chemotherapy in combination with an antigen. As described in Example II, paclitaxel increased the humoral response against ovalbumin. The testing of paclitaxel as improving a whole-cell vaccine in mice was performed as shown in Examples III and V. This principle is illustrated in Fig. 1. Sterilizing cancer cells with chemotherapeutic agents before immunization of the mice with a composition of cancer cells undergoing apoptosis and/or necrosis and chemotherapeutic agents protected the animals against a lethal tumor challenge. This was unexpected since chemotherapeutic agents are known to be toxic and to trigger systemic immunosuppression. Also, to have in one single composition a chemotherapeutic agent(s) and an antigen administered simultaneously simplifies the use of chemotherapeutic agents as potential vaccine adjuvants. The presence of the chemotherapeutic agent in the anti-cancer vaccine composition also renders moot or diminish the need to proceed to the inactivation by irradiation of the tumoral cells forming the anti-cancer vaccine before reinoculation. The drugs, especially paclitaxel, helps to raise an immune response by inducing a variety of genes known to be antigenic. In the case of melanoma cells, for which it is difficult to treat because they are known to be resistant to chemotherapy, these antigenic genes are usually differentiation genes known to play a role in the production of pigments in the skin. Melanine levels in B16 cells pretreated with paclitaxel increases and seems to be correlated with immunogenicity of the cells. Furthermore we noticed that doses of paclitaxel used to sterilize B16 cells *in vitro* do not correlated with the same doses used to immunized. Time of exposure and doses of paclitaxel needed to assure the sterilization *in vivo* are usually higher than what observed *in vitro*. This has some implications to avoid the reinoculation of cells in a distant injection site of a patient. If not property sterilized with the drugs, cancer cells may grow in a second site which is not desired.

Based on the Examples provided in this application, more particularly Examples V and VI, it is shown that chemotherapeutic agents can act as adjuvant in virtually any kind of antigenic compositions.

The chemotherapeutic agents that are intended to be included in the present application are any chemotherapeutic agents having an immunomodulator effect and/or eliciting an immune response from a patient, possibly due to their structural effect on the cell's cytoskeleton and/or microtubules. Examples of these chemotherapeutic agents include, but are not limited to, taxanes, Paclitaxel (Taxol™), cisplatine, doxorubicin, cyclophosphamide and 5-fluorouracil.

The compositions of the invention also comprise the use of various drug delivery systems or formulations. These could further improve the adjuvant property or a depot of the chemotherapeutic agents described above. The chemotherapeutic agents could be formulated in a form selected but not limited to the group consisting of a liquid solution, a powder, a polymer system, a biopolymer and natural polymer, a microparticle, a bioadhesive polymer, needleless delivery system, a scarification delivery system, and a tyne delivery system or formulated with any known and newly developed drug delivery systems (see Example IX)

Beside whole-cell vaccine, the type of vaccines that can be potentially be improved and benefit from chemotherapeutic agents-based adjuvants are DNA vaccine, conventional vaccines (protein and peptide-based), attenuated and live viruses or with already existing selected vaccine from the group consisting of but not limited to, EGF, Anti-idiotypic cancer vaccines, Gp75 antigen, GMK melanoma vaccine, MGV ganglioside conjugate vaccine, Her2/neu, Ovarex, M-Vax, O-Vax, L-Vax, STn-KHL theratope, BLP25 (MUC-1), liposomal idiotypic vaccine, Melacine, peptide antigen vaccines, toxin/antigen vaccines, MVA-based vaccine, PACIS, BCG vacine, TA-HPV, TA-CIN, DISC-virus and ImmuCyst/TheraCys. Rituxan, IDEC-C2B8, anti-CD20 Mab, Panorex, 3622W94, anti-EGP40 (17-1A) pancarcinoma antigen on adenocarcinomas Herceptin, anti-Her2, Anti-EGFr, BEC2, anti-idiotypic-GD.sub.3 epitope, Ovarex, B43.13, anti-idiotypic CA125, 4B5, Anti-VEGF, RhuMAb, MDX-210, anti-HER-2, MDX-22, MDX-220, MDX-447, MDX-260, anti-GD-2, Quadramet, CYT-424, IDEC-Y2B8, Oncolym, Lym-1, SMART M195, ATRAGEN, LDP-03, anti-CAMPATH, ior t6, anti CD6, MDX-11, OV103, Zenapax, Anti-Tac, anti-IL-2 receptor, MELIMMUNE-2, MELIMMUNE-1, CEACIDE, Pretarget, NovoMAb-G2, TNT, anti-histone, Gliomab-H, GNI-250, EMD-72000, LymphoCide, CMA 676, Monopharm-C, ior egf/r3, ior c5, anti-FLK-2, SMART 1D10, SMART ABL 364, and ImmuRAIT-CEA.

Preferably the vaccine formulations will contain the adjuvant compositions of the present invention and an antigen or antigenic composition capable of eliciting an immune response against a human or animal pathogen. Antigen or antigenic compositions known in the art can be used in the compositions of the invention, including polysaccharide antigens, antigen or antigenic compositions derived from HIV-1, (such as gp120 or gp160), any of Feline Immunodeficiency virus, human or animal herpes viruses, such as gD or derivatives thereof or Immediate Early protein such as ICP27 from HSV1 or HSV2, cytomegalovirus (especially human) (such as gB or derivatives thereof), Varicella Zoster Virus (such as gpl, II or III), or from a hepatitis virus such as hepatitis B virus for example Hepatitis B Surface antigen or a derivative thereof, hepatitis A virus, hepatitis C virus and hepatitis E virus, or from other viral pathogens, such as Respiratory Syncytial virus (for example HSRV F and G proteins or immunogenic fragments thereof disclosed in U.S. Pat. No. 5,149,650 or chimeric polypeptides containing immunogenic fragments from HSRV proteins F and G, eg FG glycoprotein disclosed in U.S. Pat. No. 5,194,595), antigens derived from meningitis strains such as meningitis A, B and C, *Streptoccoccus Pneumonia*, human papilloma virus, *Influenza* virus, *Haemophilus Influenza B* (Hib), Epstein Barr Virus (EBV), or derived from bacterial pathogens such as *Salmonella, Neisseria, Borrelia* (for example OspA or OspB or derivatives thereof), or *Chlamydia*, or *Bordetella* for example P.69, PT and FHA, or derived from parasites such as plasmodium or toxoplasma.)

In order to further improve the efficacy of the type of vaccines and compositions described above (vaccines adjuvanted with chemotherapeutic agents) can be combined before or after their administration with other therapeutic agents like 1) cytokines, 2) antibodies either monoclonal or polyclonal, 3) systemic chemotherapeutic agents (Example VII), 4) biological response modifiers including but not limited to interferon, and lymphokines such as IL-2, 5) hormone replacement therapy including but not limited to tamoxifen alone or in combination with progesterone. In addition, in order to further improve the efficacy of the type of vaccines and compositions described above (vaccines adjuvanted with chemotherapeutic agents) can be combined before or after their administration with other treatment modalities like 1) radiotherapy and 2) debulking surgery.

Other known adjuvants may be used before, after, simultaneously with the anti-cancer vaccine composition of the present invention which includes chemotherapeutic adjuvants (Example IX). The adjuvants are selected from, but not limited to, the group consisting of CpG nucleic acids, MF59, SAF, MPL, BCG and QS21 or selected from the group consisting of adjuvants that create a depot effect, adjuvants that stimulate the immune system, and adjuvants that create a depot effect and stimulate the immune system and mucosal adjuvants (alum; emulsion-based formulations including mineral oil, non-mineral oil, water-in-oil or oil-in-water-in oil emulsion, oil-in-water emulsions such as Seppic ISA series of Montanide adjuvants; PROVAX, saponins purified from the bark of the Q. saponaria tree; poly[di(carboxylatophenoxy)phosph- azene; derivatives of lipopolysaccharides, muramyl dipeptide and threonyl-muramyl dipeptide; OM-174; and *Leishmania* elongation factor, ISCOMs; SB-AS2; SB-AS4; non-ionic block copolymers that form micelles such as CRL 1005; and Syntex Adjuvant Formulation. Mucosal adjuvants are selected from the group consisting of CpG nucleic acids, Bacterial toxins, Cholera toxin, CT derivatives, CT B subunit; CTD53; CTK97; CTK104; CTD53/K63; CTH54; CTN107; CTE114; CTE112K; CTS61F; CTS106; and CTK63, Zonula occludens toxin, zot, *Escherichia coli* heat-labile enterotoxin, Labile Toxin, LT derivatives, LT B subunit; LT7K; LT61F; LT112K; LT118E; LT146E; LT192G; LTK63; and LTR72, Pertussis toxin, PT-9K/129G; Toxin derivatives; Lipid A derivatives, MDP derivatives; Bacterial outer membrane proteins, outer surface protein A (OspA) lipoprotein of *Borrelia burgdorferi*, outer membrane protein of *Neisseria meningitidis*; Oil-in-water emulsions, Aluminum salts; and Saponins, ISCOMs, the Seppic ISA series of Montanide adjuvants, Montanide ISA 720; PROVAX; Syntext Adjuvant Formulation; poly[di(carboxylatophenoxy) phosphazene and *Leishmania* elongation factor.

One category of subjects intended for treatment according to the methods of the invention and the compositions include those having a cancer or are at risk of developing a cancer selected from the group consisting of basal cell carcinoma, bladder cancer, bone cancer, brain and CNS cancer, breast cancer, cervical cancer, colon and rectum cancer, connective tissue cancer, esophageal cancer, eye cancer, kidney cancer, larynx cancer, liver cancer, lung cancer, Hodgkin's lymphoma, Non-Hodgkin's lymphoma, melanoma, myeloma, leukemia, oral cavity cancer (e.g., lip, tongue, mouth, and pharynx), ovarian cancer, pancreatic cancer, prostate cancer, rhabdomyosarcoma, skin cancer, stomach cancer, testicular cancer, and uterine cancer. In preferred embodiments, the cancer to be treated may be selected from the group consisting of esophageal cancer, eye cancer, larynx cancer, oral cavity cancer (e.g., lip, tongue, mouth, and pharynx), skin cancer, cervical cancer, colon and rectum cancer, melanoma, stomach cancer, and uterine cancer.

The present invention will be more readily understood by referring to the following examples which are given to illustrate the invention rather than to limit its scope.

### Example I

### Paclitaxel induces MCP-1 in muscle tissue

Mice are injected intramuscularly (bilaterally) with varying doses of paclitaxel (100µM to 0.01 µM). Doses of paclitaxel are prepared by 10-fold serial dilutions, in saline, of a 10mM DMSO stock. The muscles are harvested, on dry ice, 6 hours following injection, and frozen at -80 °C. RNA is isolated using TRIZOL reagent (Gibco) as per manufacturers specifications. Ten micrograms of total RNA is reverse transcribed using Superscript RT (Gibco), 500ng oligodT primers (Gibco), and 250ng Random Hexamer primers (Gibco) for 20 minutes at room temperature, followed by 2 hours at 42°C.

MCP-1 is amplified from 2µl of reverse transcription reaction using Titanium PCR kit (Clontech). Amplification primers are as follows:
MCP-1 forward 93-114 AGGTCCCTGTCATGCTTCTGGG SEQ ID NO:1
MCP-1 reverse 490-467 GGTTGTGGAAAAGGTAGTGGATGC SEQ ID NO:2

The PCR reaction is performed for 30 cycles using 100pmole for each primer, with an annealing temperature of 68 degrees. The PCR products are analyzed on a 1.5% agarose gel, and have an expected size of 397bps.

**Table 1**

| **RT-PCR results** | |
|---|---|
| Conditions | Fold-induction over GAPDH |
| Paclitaxel (0.01, 1, 100 µM per injection) | 2, 3, 6 |
| Plasmid DNA (50 µg per injection) | 4 |
| LPS (10µg per injection) | 3 |

The data are showing that paclitaxel can induce MCP-1 production to the same extent than CpG (plasmid DNA and LPS) and therefore showing that local inflammation is taking place in the muscle.

### Example II

### Paclitaxel increase humoral response against ovalbumin

The animals are injected intramuscularly into *tibialis anterior* muscles with 50 µl containing either 1 µg of ovalbumin formulated with or without paclitaxel at 10E⁻² molar. Mice are sacrificed at various time points to perform the ELISA (2 weeks post-immunization). Ovalbumin-specific antibody responses are quantified by enzyme-linked immunosorbent assay (ELISA). Microtiter plates are coated overnight with 1 µg per well of ovalbumin. Plates are then washed once with PBS-Tween 0.01% and blocked for 2 hours at 37°C with PBS+1%BSA. The plates are then incubated with serial dilutions of mouse sera for 1 hour at 37°C. Plates are washed twice and subsequently incubated with peroxidase-conjugated goat anti-mouse IgG heavy and light chain antiserum (1:1000 in PBS-BSA 1%, Sigma #A-8924). Bound antibody is detected by incubation with ABTS substrate (Sigma #A-9941), followed by determination of absorbance at 405 nm. Anti-ovalbumine antibody levels are defined as the limiting dilution titer of serum producing an A405 greater than 0.2. The data are expressed as the percentage of responding mice and as the mean titers of responding mice.

The data shows the following titers after 2 weeks:

**Table 2**

| **Groups** | **% seroconversion (titers)** |
|---|---|
| Ovalbumin | 20% (1:20) |
| Ovalbumin + paclitaxel (1X10⁻² M) | 90% (1:1500) |
| Ovalbumin + doxorubicin (1X10⁻⁶ M) | 25% (1:100) |
| Ovalbumin + cisplatin (1X10⁻⁴ M) | 40% (1:150) |
| Ovalbumin + cyclophosphamide (1X10⁻⁵M) | 50% (1:800) |

### Example III

### Paclitaxel stimulates immune response and antitumor response against cancer cells.

Lewis Lung carcinoma cells (3LL) are cultured in RPMI supplemented with 10% FBS and penicillin and streptomycin in 5% CO₂. Parental 3LL cells are used in tumor challenge experiments. The cells for the challenge are administered by either the intravenous (i.v.) or subcutaneous (s.c.) route on both flanks, at a dose of 5x10⁵ cells/mouse. Survival is monitored for mice challenged by the i.v. route. C57BI/6 (6-8-week-old females) are used throughout this study. The animals are kept in groups of 4 and fed *ad libidum*. Before each intramuscular injection, the animals are anesthetized with a mixed solution of ketamine/xylazine (10:1 w:w) at a concentration of 110 mg/Kg. The animals are injected intramuscularly into *tibialis anterior* muscles with 50 µl containing either 10E⁵ cells with or without paclitaxel (10E⁻³ M) dissolved in DMSO. Mice are immunized on the same day than the tumor challenge. Mice are sacrificed when the tumors are getting close to a non-humane size (Institutional guidelines are followed). The results show that there is a tumor protection (40% survival) following a lethal tumor challenge only in the groups immunized with 3LL cells sterilized with paclitaxel while the animals immunized with 3LL alone all developed considerable tumors (0% survival).

### Example IV

### Histology on muscles injected with chemotherapeutic agents

Paclitaxel (PTX) (Bristol-Myers Squibb, Princeton, NJ), Doxorubicin (DOX) (Gensia, Irvine, CA), and Cisplatin (CIS) (Bristol-Myers Squibb) are diluted before injection. Cyclophosphamide (CTX) (Bristol-Myers Squibb) is diluted in sterile water before injection. PTX, CTX, Dox and CIS are injected intramuscularly (i.m.). (50µl containing various concentrations of chemotherapeutic agents). Muscles are harvested and sectioned to perform histological analysis to assess tissue damage. After 7 days, no sign of tissue damage is observed, only mild to moderate infiltration of lymphocytes is observed which is in correlation with normal inflammation caused by the needle track.

### Example V

### PTX, CTX, Dox and CIS improve whole-cell vaccine.

PTX (Bristol-Myers Squibb, Princeton, NJ), DOX (Gensia, Irvine, CA), and CIS (Bristol-Myers Squibb) are diluted before injection. CTX (Bristol-Myers Squibb) is diluted in sterile water before injection. PTX, CTX, Dox and CIS are injected i.m. in combination with the whole-cell vaccine (B16 cells). On the day of vaccination, vaccine cells grown *in vitro* are trypsinized, washed three times in HBSS (pH 7.4; Life Technologies, Inc.), and counted. The cells are resuspended in HBSS at 107 cells/ml and sterilized with PTX, CTX, DOX and CIS. Eight-week-old C57BI/6 mice are given three simultaneous 100-µl s.c. injections (right and left hind limbs and left arm) using a 1-ml tuberculin syringe with a 27-gauge needle. The mice in the vaccine group receive three simultaneous injections of 106 B16 cells. Tumor occurrence (shown as the tumor-free probability) or changes in tumor growth are monitored twice a week. Changes in tumor growth (mm²) are determined by multiplying the two perpendicular diameters.

**Table 3**

| **Groups immunized i.m.** | **Average time for the tumor to reach 1 cm³ (Days)** |
|---|---|
| Non-treated | 7 |
| B16 + Gamma-irradiated (75 Gray) | 11 |
| B16 + paclitaxel (1X10⁻⁵M) | 20 |
| B16 + doxorubin (1x10⁻⁵M) | 6 |
| B16 + cyclophosphamide (1X10⁻⁵M) | 15 |
| B16 + cisplatin (1X10⁻⁵M) | 13 |

The experiment shows that B16 cells killed with PTX, CTX, DOX, and CIS generate a significant delay of tumor growth as opposed to B16 cells sterilized by standard irradiation protocols. Compared to whole-cell vaccines killed by standard irradiation, the delay of tumor growth has doubled in mice immunized with B16 cells killed by chemotherapeutic agents. This data show that chemotherapeutic agent-based vaccines are better than irradiated whole-cell vaccines.

### Example VI

### Using PTX as adjuvants to improve DNA-based tumor vaccination

Melanoma is generally resistant to chemotherapy and radiation therapy. Its unique immunological properties give support for developing innovative new therapies via manipulation of the patient's own immune system. These new therapies use of whole-cell-based tumour vaccines, including autologous, whole-cell allogeneic and cytokine gene-modified vaccines, as well as tumour lysate vaccines, for active specific immunotherapy of melanoma. The stimulatory effect of CpG sequences in the plasmid DNA promotes the Th1 (cellular) immune response observed in DNA vaccines. It is possible to combine this effect with the described immune stimulatory effect of PTX in an anti-tumor DNA vaccination protocol. Briefly, plasmid encoding MAGE or β-galactosidase (negative control), 50µg in 50 µl saline is injected intramuscularly into 6-8 week-old female C57/BI mice alone or with varying doses of PTX (1µM). Injections are performed twice at 2-week intervals, the initial injection at Day 0 and then at Day 14 alternatively into the anterior tibialis of the left and right legs. The vaccination protocol is tested in *in vivo* tumor models: for survival following i.v. challenge with B16 melanoma cells. In the survival assay, 2x10⁵ B16 melanoma tumor cells are injected intravenously to mice vaccinated with either MAGE plasmid or β-galactosidase plasmid (as above), Day 14 after immunizations. Each group consisted in 10 mice. This protocol is performed to evaluate survival of vaccinated animals (MAGE) versus negative control (β-galactosidase) animals. Survival is scored as percent surviving mice over time (days).

**Table 4**

| **Groups** | **Survival at day 40 (%)** |
|---|---|
| untreated | 0 |
| Immunized with Bgal | 10 |
| Immunized with MAGE + paclitaxel (100 µM) | 50 |
| Immunized with MAGE alone | 30 |

The experiment showed that the mice immunized with the plasmid encoding MAGE formulated with chemotherapeutic agents survived the longest as compared to untreated mice and mice treated with non formulated naked plasmid DNA or treated with a non-relevant antigen like β-galactosidase. The factor of improvement is at least 2- to 3-fold better.

### Example VII

### Use of other adjuvant or drug delivery system to improve further chemotherapeutic agent-based vaccine

The experiment described in Example III is repeated using exopolysaccharide-formulated paclitaxel (Profilactis™). The formulation is used to deliver paclitaxel and to sterilize.. The microspheres are generated by spray-drying and used to deliver paclitaxel and to sterilize the cancer cells before immunization. Compared to DMSO, exopolysaccharide-formulated paclitaxel is more efficient to enhance survival of mice challenged with cancer cells. All mice survived a lethal tumor challenged.

### Example VIII

### Evaluation of the paclitaxel toxicity on C2C12 cells

C1C12 cells were plated in a 12-wells plate at 7X10⁴ cells/ml/well in complete DMEM (10 FBS, 1% Strepto-pen, 1% Hepes) and incubated O/N at 37°C under 5% CO2 atmosphere. Twenty-four hours later, normal DMEM was changed for DMEM containing 2% Horse serum, 1% Strepto-pen and 1% Hepes. The medium was then changed everyday with the complete DMEM 2% Horse serum. For an extra 7 days, complete DMEM 10% Horse serum was added to the cells. After about 1 week, all cells were differentiated and treated with various concentrations of paclitaxel. Even at a high concentration of paclitaxel, differentiated cells now forming elongated muscular fibers, were not affected by any treatment probably due to the non-dividing state of the cells. This observation support the fact that drugs can be injected in the muscle without necessarily causing damages or cell death.

### Example IX

### Prophylactic immunization with cancer cells pretreated with paclitaxel or γ-irradiated

Tumor cells were trypsinized and washed 3 times with PBS. Cells were gamma-imadiated with 75 Gy using a Cs¹³⁷ source and injected i.m. or kept in culture for further in vitro analysis. For induction of apoptosis (cell death), tumor cells were exposed to Profilactis™ (paclitaxel 100µM + 0.1% exopolysaccharides) for 4 hours before being used for immunization. Complete cell death was confirmed by trypan blue staining before injection and subsequent cell culturing. Ten mice were vaccinated twice (days 0 and 14) with B16 cells pretreated with Profilactis™ and with γ-irradiated cells. After 3 weeks, mice were challenged with 1X10⁶ viable B16 tumor cells. Mice were monitored for survival and presence of tumors over time.

**Table 5**

| **Tumor-free mice over time (%)** | | | | |
|---|---|---|---|---|
| Days | 1x10⁴ cells pretreated with Paclitaxel | 1X10⁵ cells treated with Paclitaxel | 1X10⁴ γ-irradiated cells | 1X10⁵ γ-irradiated cells |
| 20 | 100 | 100 | 100 | 100 |
| 40 | 100 | 100 | 60 | 80 |
| 80 | 100 | 100 | 20 | 40 |
| 160 | 80 | 100 | 0 | 0 |

### Example X

### Therapeutic immunization with cancer cells pretreated with paclitaxel or γ-irradiated

Tumor cells were trypsinized and washed 3 times with PBS. Cells were gamma-irradiated with 75 Gy using a Cs¹³⁷ source and injected i.m. or kept in culture for further in vitro analysis. For induction of apoptosis (cell death), tumor cells were exposed to Profilactis™ (paclitaxel 100µM + 0.1% exopolysaccharides) for 4 hours before being used for immunization. Complete cell death was confirmed by trypan blue staining before injection and subsequent cell culturing. Ten mice were challenged (i.v.) with 1x10⁶ viable B16 tumor cells on day 0 and treated on day 7 with B16 cells pretreated with Profilactis™ and with γ-irradiated cells. Mice were monitored for survival and presence of tumors over time.

**Table 6**

| **Tumor-free mice over time (%)** | | | | |
|---|---|---|---|---|
| Days | 1x10⁴ cells pretreated with Paclitaxel | 1X10⁵ cells treated with Paclitaxel | 1X10⁴ γ-irradiated cells | 1X10⁵ γ-irradiated cells |
| 20 | 100 | 100 | 70 | 100 |
| 40 | 100 | 100 | 0 | 50 |
| 80 | 70 | 100 | 0 | 0 |
| 160 | 60 | 70 | 0 | 0 |

### Example XI

### Prophylactic immunization with cancer cells pretreated with paclitaxel or γ-irradlated in cyclophosphamide-immunocompromised mice

Tumor cells were trypsinized and washed 3 times with PBS. Cells were gamma-irradiated with 75 Gy using a Cs¹³⁷ source and injected i.m. or kept in culture for further in vitro analysis. For induction of apoptosis (cell death), tumor cells were exposed to Profilactis™ (paclitaxel 100µM + 0.1% exopolysaccharides) for 4 hours before being used for immunization. Complete cell death was confirmed by trypan blue staining before injection and subsequent cell culturing. In one group, cyclophosphamide was used at 100µg/ml injected 3 times a week by the I.P. route to induce immunosuppression as measured by an hematocrit evaluation. Ten mice were vaccinated twice (days 0 and 14) with B16 cells pretreated with Profilactis™, with γ-irradiated cells and with γ-irradiated cells formulated with MPL. After 3 weeks, mice were challenged with 1x10⁶ viable B16 tumor cells. Mice were monitored for survival and presence of tumors over time.

**Table 7**

| **Tumor-free mice over time (%)** | | | | |
|---|---|---|---|---|
| Days | 1x10⁵ cells pretreated with Profilactis™ | 1X10⁵ cells pretreated with Profllactis™ but in cyclophosphamide-immunocompromised mice | 1X10⁵ γ-irradiated cells | 1X10⁵ γ-irradiated cells and formulated with MPL |
| 20 | 100 | 100 | 100 | 100 |
| 40 | 100 | 100 | 70 | 60 |
| 80 | 100 | 70 | 30 | 20 |
| 160 | 80 | 50 | 0 | 0 |

## Claims

1. An anti-cancer vaccine composition comprising an antigen in association with an effective amount of at least one immunomodulator chemotherapeutic adjuvant eliciting an immune response in a patient and a pharmaceuticallly acceptable carrier wherein said chemotherapeutic adjuvant is a taxane.

2. The anti-cancer vaccine of claim 1, wherein said vaccine is selected from the group consisting of whole-cell vaccine, DNA vaccine, protein-based vaccine, peptide-based vaccine and attenuated organism vaccine.

3. The anti-cancer vaccine of claim 1, wherein said antigen is inactivated tumor cells.

4. The anti-cancer vaccine of claim 1, wherein said antigen is tumor cells inactivated by said chemotherapeutic agent.

5. The anti-cancer vaccine of claim 3, wherein said tumor cells are inactivated by radiotherapy and/or chemotherapy.

6. The anti-cancer vaccine of claim 3, wherein said tumor cells are inactivated by radiotherapy.

7. The anti-cancer vaccine of claim 1, wherein said taxane is Paclitaxel.

8. The anti-cancer vaccine of any one of claims 1-7, further comprising a therapeutically effective amount of a therapeutic agent.

9. The anti-cancer vaccine of claim 8, wherein said therapeutic agent is selected from the group consisting of cytokine, antibody, systemic chemotherapeutic agent, biological response modifier and hormones.

10. The anti-cancer vaccine of claim 9, wherein said antibody is a monoclonal antibody.

11. The anti-cancer vaccine of claim 9, wherein said biological response modifier is selected from the group consisting of interferon and lymphokine.

12. The anti-cancer vaccine of claim 11, wherein said lymphokine is IL-2.

13. The anti-cancer vaccine of any one of claims 1-12, further comprising a non-chemotherapeutic adjuvant selected from the group consisting of polysaccharides, CpG nucleic acids, MF59, SAF, MPL and QS21.

14. The anti-cancer vaccine of any one of claims 1-13, further comprising a non-chemotherapeutic adjuvant selected from the group consisting of adjuvants that create a local reservoir of drug, adjuvants that stimulate the immune system, mucosal adjuvants, phosphazene and *Leishmania* elongation factor.

## Patentansprüche

1. Antikrebs-Impfstoffzusammensetzung, welche ein Antigen in Verbindung mit einer wirksamen Menge von mindestens einem immunomodulatorischen chemotherapeutischen Adjuvans, durch das bei einem Patienten eine Immunantwort ausgelöst wird, sowie einem pharmazeutisch zulässigen Carrier umfasst, wobei es sich bei dem chemotherapeutischen Adjuvans um ein Taxan handelt.

2. Antikrebs-Impfstoff gemäß Anspruch 1, wobei der Impfstoff aus einer Gruppe ausgewählt wird, zu der Ganzzell-Impfstoff, DNA-Impfstoff, Protein-basierter Impfstoff, Peptidbasierter Impfstoff sowie auf abgeschwächten Organismen basierender Impfstoff gehören.

3. Antikrebs-Impfstoff gemäß Anspruch 1, wobei es sich bei dem Antigen um inaktivierte Tumorzellen handelt.

4. Antikrebs-Impfstoff gemäß Anspruch 1, wobei es sich bei dem Antigen um Tumorzellen handelt, welche durch den chemotherapeutischen Wirkstoff inaktiviert wurden.

5. Antikrebs-Impfstoff gemäß Anspruch 3, wobei die Tumorzellen durch Radiotherapie und/oder Chemotherapie inaktiviert wurden.

6. Antikrebs-Impfstoff gemäß Anspruch 3, wobei die Tumorzellen durch Radiotherapie inaktiviert wurden.

7. Antikrebs-Impfstoff gemäß Anspruch 1, wobei es sich bei dem Taxan um Paclitaxel handelt.

8. Antikrebs-Impfstoff gemäß einem der Ansprüche 1 bis 7, ferner eine therapeutische Menge eines therapeutischen Wirkstoffes umfassend.

9. Antikrebs-Impfstoff gemäß Anspruch 8, wobei der therapeutische Wirkstoff aus einer Gruppe ausgewählt wird, zu welcher Zytokin, Antikörper, systemischer chemotherapeutischer Wirkstoff, Modifikatoren der biologischen Antwort sowie Hormone gehören.

10. Antikrebs-Impfstoff gemäß Anspruch 9, wobei es sich bei dem Antikörper um einen monoklonalen Antikörper handelt.

11. Antikrebs-Impfstoff gemäß Anspruch 9, wobei der Modifikator der biologischen Antwort aus einer Gruppe ausgewählt wird, zu welcher Interferon und Lymphokin gehören.

12. Antikrebs-Impfstoff gemäß Anspruch 11, wobei es sich bei dem Lymphokin um IL-2 handelt.

13. Antikrebs-Impfstoff gemäß einem beliebigen der Ansprüche 1 bis 12, ferner ein nicht-chemotherapeutisches Adjuvans umfassend, welches aus einer Gruppe ausgewählt wird, zu der Polysaccharide, CpG-Nukleinsäuren, MF59, SAF, MPL und QS21 gehören.

14. Antikrebs-Impfstoff gemäß einem beliebigen der Ansprüche 1 bis 13, ferner ein nicht-chemotherapeutisches Adjuvans umfassend, das aus der Gruppe von Adjuvantien besteht, welche ein lokales Reservoir eines Medikaments erzeugen, Adjuvantien, welche das Immunsystem stimulieren, mukosalen Adjuvantien, Phosphazen und Leishmania-Verlängerungsfaktor gehören.

## Revendications

1. Composition vaccinale anti-cancer comportant un antigène en association avec une quantité efficace d'au moins un adjuvant chimio-thérapeutique immunomodulateur provoquant une réponse immunitaire chez un patient et un véhicule pharmaceutiquement acceptable, dans laquelle ledit adjuvant chimio-thérapeutique est une taxane.

2. Vaccin anti-cancer selon la revendication 1, dans lequel ledit vaccin est choisi dans le groupe constitué d'un vaccin à base de cellule entière, de vaccin à ADN, de vaccin à base de protéine, de vaccin à base de peptide et de vaccin à base d'organisme atténué.

3. Vaccin anti-cancer selon la revendication 1 dans lequel ledit antigène est fait de cellules de tumeur inactivées.

4. Vaccin anti-cancer selon la revendication 1, dans lequel ledit antigène est fait de cellules de tumeur inactivées par ledit agent chimio-thérapeutique.

5. Vaccin anti-cancer selon la revendication 3, dans lequel lesdites cellules tumorales sont inactivées par radiothérapie et/ou chimiothérapie.

6. Vaccin anti-cancer selon la revendication 3, dans lequel lesdites cellules tumorales sont inactivées par radiothérapie.

7. Vaccin anti-cancer selon la revendication 1, dans lequel ladite taxane est du Paclitaxel.

8. Vaccin anti-cancer selon l'une quelconque des revendications 1 à 7, comportant, de plus, une quantité thérapeutiquement efficace d'un agent thérapeutique.

9. Vaccin anti-cancer selon la revendication 8, dans lequel ledit agent thérapeutique est choisi dans le groupe constitué de cytokine, d'anticorps, d'agent chimiothérapeutique systémique, d'un modificateur de réponse biologique et d'hormones.

10. Vaccin anti-cancer selon la revendication 9, dans lequel ledit anticorps est un anticorps monoclonal.

11. Vaccin anti-cancer selon la revendication 9, dans lequel ledit modificateur de réponse biologique est sélectionné à partir du groupe constitué d'interféron et de lymphokine.

12. Vaccin anti-cancer selon la revendication 11, dans lequel ladite lymphokine est IL-2.

13. Vaccin anti-cancer selon l'une quelconque des revendications 1 à 12 comportant, de plus, un adjuvant non chimio-thérapeutique choisi dans le groupe constitué de polysaccharides, d'acides nucléiques CpG, de MF59, de SAUF, de MPL et de QS21.

14. Vaccin anti-cancer selon l'une quelconque des revendications 1 à 13, comprenant, de plus, un adjuvant non chimio-thérapeutique choisi dans le groupe constitué d'adjuvants qui créent un réservoir local de médicament, d'adjuvants qui stimulent le système immunitaire, d'adjuvant par voie muqueuse, de phosphazène et de facteur d'élongation de *Leishmania*.


### SEQUENCE LISTING

<110> Simard, Eric
   Lemieux, Pierre
   Goyette, Philippe
   Technologies Biolactis Inc.
<120> Chemotherapeutic agents as anti-cancer
   vaccine adjuvants and therapeutic methods thereof
<130> 15465-3PCT
<150> US 60/366,531
   <151> 2002-03-25
<160> 2
<170> FastSEQ for Windows Version 4.0
<210> 1
   <211> 22
   <212> DNA
   <213> Artificial Sequence
<220>
   <223> primer: MCP-1 forward 93-114
<400> 1
   aggtccctgt catgcttctg gg 22
<210> 2
   <211> 24
   <212> DNA
   <213> Artificial Sequence
<220>
   <223> primer: MCP-1 reverse 490-467
<400> 2
   ggttgtggaa aaggtagtgg atgc 24